# EUROPEAN PATENT APPLICATION

(11) **EP 3 424 448 A1**
(43) Date of publication of application: **09.01.2019**
(21) Application number: 17425073.8
(22) Date of filing: 07.07.2017
(51) Int. Cl.: A61B 17/34, A61B 17/12, A61B 17/00, A61B 17/22, A61M 25/09, A61B 17/11

(54) **SURGICAL SYSTEM FOR CONNECTING VASCULAR SEGMENTS THAT ARE NOT FUNCTIONALLY CONTIGUOUS**

(71) Applicant: Clementi, Fabrizio, 00166 Roma (IT); Corvo, Pierfrancesco, 00166 Roma (IT)
(72) Inventor: Clementi, Fabrizio, 00166 Roma (IT); Corvo, Pierfrancesco, 00166 Roma (IT)

(57) **Abstract**

Surgical system and related by-pass process connecting vascular segments that are not functionally contiguous, comprising:
- a first endovascular catheter (207), inserted under fluoroscopic guidance in the first artery (203) that has to be connected - i.e. the left anterior descending artery (LAD); said first catheter (207) comprising a hollow needle and a thin metallic guiding wire (211) having few tenths of a millimeter thickness, able to puncture and pass through the walls of the arteries;
- a second endovascular catheter (208), inserted under fluoroscopic guidance in the second artery (204) that has to be connected - i.e. the left internal mammary artery (LIMA); said second catheter (208) comprising a hollow needle and a thin metallic guiding wire (212) having few tenths of a millimeter thickness, able to puncture and pass through the walls of the arteries;
characterized in that:
- the distal ends of both the guiding wires (211) and (212) comprise some connection means, represented in example by magnetized elements, so that they are attracted and connected each other in order to compose a unique wire,
so that one of the two guiding wires, i.e. the guiding wire (212), can be pulled and extracted completely, pulling the distal end of the other guiding wire (211), so that only the latter one is kept, and it achieves a unique track in respect of which, once the catheters (207) and (208) have been removed, it is possible to insert and install a stent (213) inside the vascular system; said stent (213), properly shaped, has geometric characteristics that achieve a complete haemostasis of the arterial walls, and the whole system represents a complete vascular by-pass between the left anterior descending artery (LAD) (203) and the left internal mammary artery (LIMA) (204).

## Description

The present invention is related essentially to a surgical system including a catheter with balloon and needle, some guiding wires and a vascular stent properly shaped. The system permits to achieve, working through endovascular ways only, a by-pass connecting vascular segments that are not functionally contiguous. In such a way the blood can overcome an obstruction that is occurred inside a blood vessel.

As known, the aortocoronary by-pass has always been considered a solution in the treatment of the three-vessels coronary diseases (that is when all the three coronary branches are involved), either in case of serious or mild diseases. That happens although in recent years the stents technology, and especially the drug eluting stents technology, has increased significantly the safety and the effectiveness of these devices in the treatment of the atherosclerotic coronary disease. With reference to that, also the most recent clinic trials that compare the two techniques of revascularization have demonstrated as the aortocoronary by-pass results to be significantly better, with reference either to decrease of mortality and to decrease of the risk of heart attack, yet after five years since the treatment with increase of the benefit according to the passing of time.
It is widely demonstrated that, the significant advantage of the traditional surgery, in respect to the endovascular techniques, arises especially from the use of the internal mammary artery (IMA) as the vessel chosen for the revascularization of the left anterior descending artery (LAD). Indeed, the IMA presents a low risk of obstruction and atherosclerosis due to the local characteristics keeping an open flow in the long term, even compared to the vessel grafts (SVG). That achieves a good flow of oxygenated blood in the area of distribution of the anterior descending artery (LAD), with significant perfusion pressures, even in case of serious disease in the receiving vessel. It is commonly observed that, in case of very serious events of atherosclerotic burden, the levels of coronary reserve are significantly higher when an arterious graft is installed in the internal mammary artery.
Recently, a trial using computed tomography to detect the anatomic shapes of the thoracic vascular structures has permitted to evaluate how the left internal mammary artery (LIMA) is very close to the anterior descending artery (LDA). Indeed, in all the analyzed patients there is a point in the chest where the two arteries cross by, being separated by just few millimeters of serous tissue. Furthermore, since the beginning of the past century it is well know the presence of natural by-passes connecting the internal mammary artery to the coronary artery through the pericardiacophrenic branch. The presence of these anastomosis has been indeed the rational of the surgical connection of the IMA before the introduction of the aortocoronary by-pass. Recently, a research has shown that in large part of patients it is possible to recruit functioning connections between the left internal mammary artery (LIMA) and the LAD, and between the right internal mammary artery (RIMA) and the right coronary (RC).

The proposed method, disclosed by the present invention, permits to achieve a by-pass between the left internal mammary artery (LIMA) and the LAD, and between the right internal mammary artery (RIMA) and the right coronary (RC), using a technique that is exclusively endoscopic, taking advantage of the close distance of the two respective vascular structures. The same technique can be used in order to achieve other by-passes in other vascular segments either.

Nowadays, the only technique able to achieve an arterial by-pass to the internal mammary artery (IMA) is the open-chest surgery, with the endovascular methods being limited to the insertion of stents in the native coronary vessels. However, the open-chest surgery is affected by high rates of morbidity, especially in some categories of patients (persons having mild or serious respiratory failure, persons having serious calcific disease of the aorta, persons having senile dementia or being of very old age).
The present endovascular technique permits the revascularization with the internal mammary artery even in persons that are now excluded from the traditional surgery, and opens new opportunities to the endovascular surgery. That decreases the post-surgery complications and does not require the deep sedation and the orotracheal intubation of the patient. It further permits a very fast recovery from the surgery, without the requirement to monitor the patient in a post-surgery intensive care unit. The absence of a deep anaesthesia further increases the number of treatable patients: big obese persons, patients affected by chronic respiratory failure or degenerative diseases like Alzheimer, or very old persons.
Therefore, this technique leads to significant advantages in respect to the open-chest surgery, either with reference to costs (decrease of the complexity of interventions with consequent decrease of the costs in the hospital, decrease of the patient's recovery time with related convalescence time and return back to work time), and possibly with reference to the morbidity.

The proposed surgery system permits, therefore, an intervention entering from a peripheric vascular access achievable by a simple arterious or venous puncture. That can be done under fluoroscopic guidance (X rays) and having the patient that is partially sedated or under local anaesthesia. In such a way, some endovascular surgery techniques known to the physicians can be used, modifying some aspects but without changing completely the basic technique.
The system is able to achieve a by-pass connecting adjacent arterious or venous segments by an exclusively endovascular way, achieving the optimal haemostasis to the patient, and permitting to the blood to reach the organs that otherwise would be affected by chronic ischaemia.
Just as examples, without reporting all the possible cases, possible vascular segments are here listed where the system could be applied:
- left coronary and left internal mammary artery
- abdominal aorta and common femoral artery
- deep femoral artery and posterior tibial artery
- radial or ulnar artery and radial vessel
- brachial artery and humeral vessel (to achieve arterious-venous fistulas).

Therefore, it is specific subject of the present invention a surgical system and related by-pass process connecting vascular segments that are not functionally contiguous, comprising:
- a first endovascular catheter, inserted under fluoroscopic guidance in the first artery that has to be connected - i.e. the left anterior descending artery (LAD); said first catheter comprising a hollow needle and a thin metallic guiding wire having few tenths of a millimeter thickness, able to puncture and pass through the walls of the arteries;
- a second endovascular catheter, inserted under fluoroscopic guidance in the second artery that has to be connected - i.e. the left internal mammary artery (LIMA); said second catheter comprising a hollow needle and a thin metallic guiding wire having few tenths of a millimeter thickness, able to puncture and pass through the walls of the arteries;
characterized in that:
- the distal ends of both the guiding wires comprise some connection means, represented in example by magnetized elements, so that they are attracted and connected each other in order to compose a unique wire,
so that one of the two guiding wires, i.e. the first guiding wire, can be pulled and extracted completely, pulling the distal end of the other guiding wire, so that only the latter one is kept, and it achieves a unique track in respect of which, once the catheters have been removed, it is possible to insert and install a stent inside the vascular system; said stent, properly shaped, has geometric characteristics that achieve a complete haemostasis of the arterial walls, and the whole system represents a complete vascular by-pass between the left anterior descending artery (LAD) and the left internal mammary artery (LIMA).

The present invention will now be described for illustrative but not limitative purposes, according to its preferred embodiments, with particular reference to figures of the enclosed drawings, wherein:
Figure 1 is a front perspective view of a heart submitted to an intervention, where in particular a by-pass has been done between the left anterior descending artery (LAD) and the left internal mammary artery (LIMA);
Figure 2 is a schematic view of a particular of the left anterior descending artery (LAD) and the left internal mammary artery (LIMA), in a first step of the process of the present invention, where two respective catheters have been inserted;
Figure 3 is a view of the same particular of Figure 2, in a second step of the process of the present invention, where some respective balloons are inflated in the distal part of the two catheters achieving the haemostasis and stability of the system catheter-artery, the ends of two respective hollow needles are passed through the walls of the respective arteries: a guiding wire is pushed forward, by passing inside the central lumen, going beyond the wall of the artery and reaching the surrounding tissues;
Figure 4 is a view of the same particular of Figure 2, in a third step of the process of the present invention, where the needles are retracted and the ends of the two metallic guiding wires are pushed forward up to contact each other and to activate some connection means;
Figure 5 is a view of the same particular of Figure 2, in a fourth step of the process of the present invention, where the balloons are deflated, one of the two guiding wires is pulled and completely extracted, pulling the end of the other guiding wire, so that only the latter one is kept, and it achieves a unique track; in case, the stability of the connection of the two wires can be assured by using some well known lasso systems;
Figure 6 is a view of the same particular of Figure 2, in a fifth step of the process of the present invention, where the catheters are progressively pulled back and removed;
Figure 7 is a view of the same particular of Figure 2, in a sixth step of the process of the present invention, where the second catheter has been completely removed;
Figure 8 is a view of the same particular of Figure 2, in a seventh step of the process of the present invention, where the first catheter has been completely removed and there is only a thin metallic guiding wire that represents a track;
Figure 9 is a view of the same particular of Figure 2, in a eighth step of the process of the present invention, where a stent is positioned and installed by using the metallic guiding wire, so that a by-pass is achieved connecting the left anterior descending artery (LAD) to the left internal mammary artery (LIMA);
Figure 10 is a view of the same particular of Figure 2, in a ninth step of the process of the present invention, where the metallic guiding wire has been completely extracted and the stent has been left installed in its position;
Figure 11 is a lateral schematic view of a catheter modified at the distal part where a radial expansion balloon has been installed;
Figure 12 is a lateral view of a particular of the distal part of the same modified catheter of figure 11;
Figure 13 is a first perspective view of a particular of the distal part of the same modified catheter of figure 11;
Figure 14 is a second perspective view of a particular of the distal part of the same modified catheter of figure 11.

It is here underlined that only few of the many conceivable embodiments of the present invention are described, which are just some specific non-limiting examples, having the possibility to describe many other embodiments based on the disclosed technical solutions of the present invention.

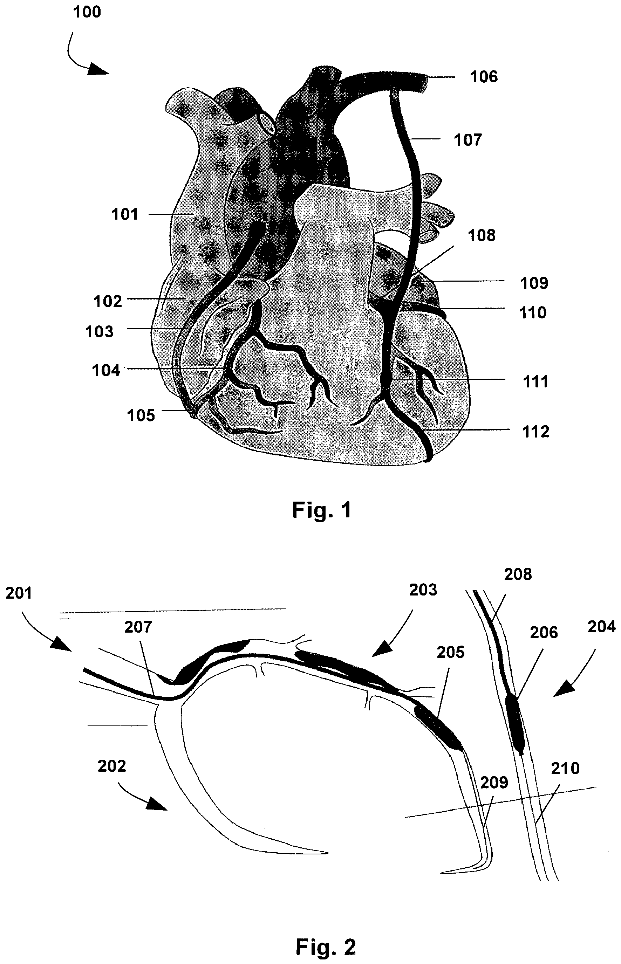

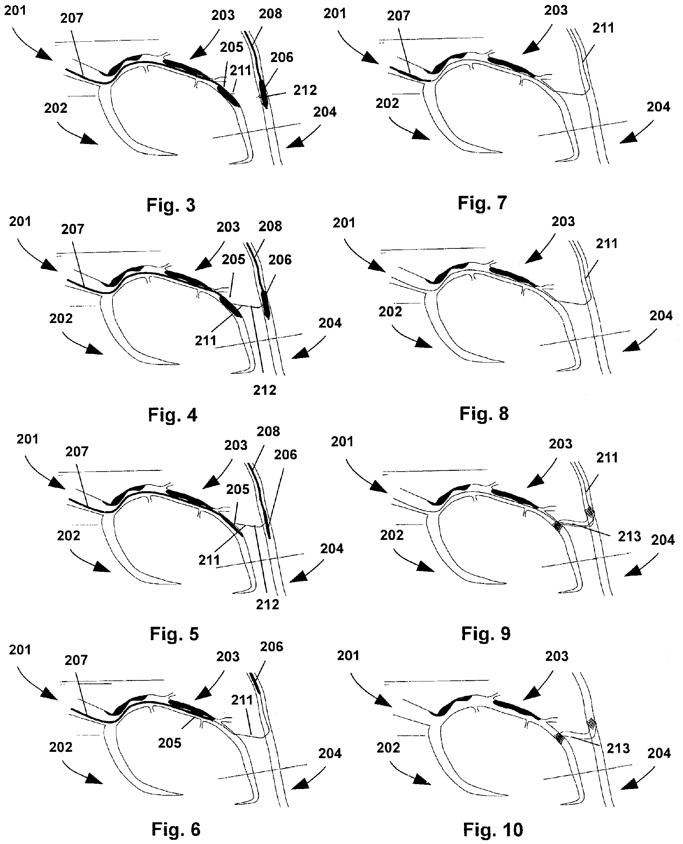

Figure 1 shows a heart submitted to a by-pass intervention. In order to explain how the surgical system of the present invention works, we can consider a by-pass between the left internal mammary artery 107 (LIMA) and the left anterior descending artery 112 (LAD).
As shown in the sequence of Figures 2 - 10, two different accesses are taken by peripheral arterial punctures. One for the catheter 208 reaching the left internal mammary artery 204 (LIMA) and the other for the catheter 207 reaching the left anterior descending artery 203 (LAD).
Both the catheters 207 and 208 comprise at their distal end a respective cylindric balloon 205, 206, that is inflated so that it can adhere to the internal walls of the artery, being hollow inside and keeping therefore the normal blood flow.
Under a fluoroscopic guidance and after a recognition by computer tomography, it is chosen the point of puncture into the mammary artery 204 (LIMA) according to the anatomical continuity between the two arterious vessels 203, 204. The system permits to provide a simultaneous puncture of the artery, using a needle having a specific and predefined length, and to make a thin metallic wire having thickness of few tenths of a millimiter to pass through the same artery. This operation is made on both the arteries 203, 204, achieving two metallic guiding wires 211, 212, having thickness of few tenths of a millimeter, with their proximal end being outside the patient close to the vascular access created at the beginning of the intervention, and their distal end in an extravascular position but close each other. The above said guiding wires 211, 212, have their distal end that can be magnetized, so that their coupling can be made easy and it can be improved by using some endovascular lassos.
Then, the surgeon can pull one or the other of the two wires, by pushing a wire 211 and pulling the other 212 from the part outside the patient, always under fluoroscopic guidance, so that a unique and reliable track is achieved, that is represented by the guiding wire 211, on which a stent 213, properly shaped, can be moved. The geometric characteristics of the stent 213 permit to create a complete haemostasis of the arterial wall in the left internal mammary artery 204 (LIMA) and in the left anterior descending artery 203 (LAD), permitting at the same time the independent moving of both the anatomical parts.
When the stent 213 has been installed, the guiding wire 211 is removed and a vascular haemostasis is provided according to the standard protocol.

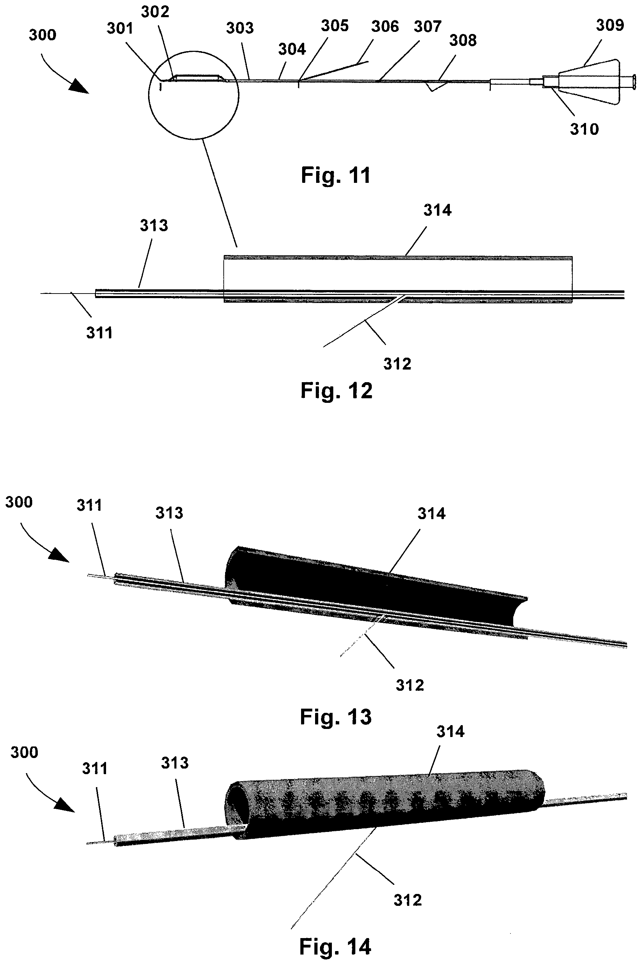

Figures 11 - 15 show a catheter 300 modified according to the principles of the present invention. In particular, the cylindric balloon 314 placed at the distal part of the catheter 300 presents a radial surface comprising knots and/or other means having a memory of shape. In such a way, when the balloon 314 is inflated, it keeps an internal hollow cavity having a perfectly cylindric shape, where a normal blood flow is assured inside. The conduit 313 is placed along a longitudinal direction and exactly at the basis of the surface in the cylindric balloon 314.
In such a way, it is possible to insert the catheter 300 by sliding it in a endovascular way, following the guiding wire 311. When the catheter 300 has reached its final position in the artery, by inflating the cylindric balloon 314 it is achieved the result to dilate and keep a sufficient stiffness into the catheter-blood vessel system, so that a proper blood flow is maintained inside the artery. In such position, in fact, it is possible to make a needle, having thickness of few tenths of a millimeter and a predefined length, able to puncture and pass through the wall of the artery.

Therefore, the present invention defines the following process to achieve a by-pass connecting vascular segments that are not functionally contiguous, using a surgical system as previously defined.
The process comprises the following steps:
- a first endovascular catheter 207 is inserted under fluoroscopic guidance in the first artery 203 that has to be connected - i.e. the left anterior descending artery (LAD); a first cylindric balloon, placed at the distal end of said first endovascular catheter 207, is inflated so that it can adhere to the internal walls of the artery 203, being hollow inside and keeping therefore the normal blood flow;
- a second endovascular catheter 208 is inserted under fluoroscopic guidance in the first artery 204 that has to be connected - i.e. the left internal mammary artery (LIMA); a second cylindric balloon, placed at the distal end of said second endovascular catheter 208, is inflated so that it can adhere to the internal walls of the artery 204, being hollow inside and keeping therefore the normal blood flow;
- a first needle goes out from the first catheter 207, at a position close to the first cylindric balloon, in order to provide a puncture and pass through the wall of the first artery; a first guiding wire having a thickness of few tenths of a millimeter is pushed going through the inner lumen of the needle, so that the first wire is in an extravascular position;
- a second needle goes out from the second catheter 208, at a position close to the second cylindric balloon, in order to provide a puncture and pass through the wall of the second artery; a second guiding wire having a thickness of few tenths of a millimeter is pushed going through the inner lumen of the needle, so that the second wire is in an extravascular position;
- the distal ends of the first and second guiding wires 211, 212 are attracted and connected each other in order to achieve a unique wire working as a track;
- one of the two guiding wires, i.e. the guiding wire 212 is pulled and completely extracted, pulling with itself the end of the other guiding wire 211, so that only the latter one is maintained, representing a track;
- both the catheters 207 and 208 are removed;
- a stent 213 is positioned and installed by using the guiding wire 211, so that a by-pass is achieved connecting the left anterior descending artery (LAD) 203 to the left internal mammary artery (LIMA) 204.

The above described examples show, therefore, that the present invention reaches all the proposed goals. In particular, it permits to achieve a by-pass connecting adjacent arterious or venous segments by an exclusively endovascular way, achieving the optimal haemostasis to the patient, and permitting to the blood to reach the organs that otherwise would be affected by chronic ischaemia.

Furthermore, the proposed system permits an intervention entering from a peripheric vascular access achievable by a simple arterious or venous puncture. That is done under fluoroscopic guidance (X rays) and having the patient that is partially sedated or under local anaesthesia. In such a way, some endovascular surgery techniques known to the physicians can be used, modifying some aspects but without changing completely the basic technique.

The present invention has been described for illustrative but not limitative purposes, according to its preferred embodiments, but it is clear that modifications and/or changes can be introduced by those skilled in the art without departing from the relevant scope, as defined in the enclosed claims.

## Claims

1. Surgical system connecting vascular segments that are not functionally contiguous, comprising:
- a first endovascular catheter (207), inserted under fluoroscopic guidance in the first artery (203) that has to be connected - i.e. the left anterior descending artery (LAD); said first catheter (207) comprising a hollow needle and a thin metallic guiding wire (211) having few tenths of a millimeter thickness, able to puncture and pass through the walls of the arteries;
- a second endovascular catheter (208), inserted under fluoroscopic guidance in the second artery (204) that has to be connected - i.e. the left internal mammary artery (LIMA); said second catheter (208) comprising a hollow needle and a thin metallic guiding wire (212) having few tenths of a millimeter thickness, able to puncture and pass through the walls of the arteries;
**characterized in that**:
- the distal ends of both the guiding wires (211) and (212) comprise some connection means, represented in example by magnetized elements, so that they are attracted and connected each other in order to compose a unique wire,
so that one of the two guiding wires, i.e. the guiding wire (212), can be pulled and extracted completely, pulling the distal end of the other guiding wire (211), so that only the latter one is kept, and it achieves a unique track in respect of which, once the catheters (207) and (208) have been removed, it is possible to insert and install a stent (213) inside the vascular system; said stent (213), properly shaped, has geometric characteristics that achieve a complete haemostasis of the arterial walls, and the whole system represents a complete vascular by-pass between the left anterior descending artery (LAD) (203) and the left internal mammary artery (LIMA) (204).

2. Surgical system connecting vascular segments that are not functionally contiguous, according to the previous claim, **characterized in that**:
- said first endovascular catheter (207) comprises at its distal end a respective cylindric balloon (205) that is inflated so that it can adhere to the internal walls of the artery (203), being hollow inside and keeping therefore the normal blood flow;
- said second endovascular catheter (208) comprises at its distal end a respective cylindric balloon (206) that is inflated so that it can adhere to the internal walls of the artery (204), being hollow inside and keeping therefore the normal blood flow.

3. Surgical system connecting vascular segments that are not functionally contiguous, according to one or more of the previous claim, **characterized in that**:
- the cylindric balloon (314) placed at the distal part of the catheter 300 presents a radial surface comprising knots and/or other means having a memory of shape, so that, when the balloon (314) is inflated, it keeps an internal hollow cavity having a perfectly cylindric shape, where a normal blood flow is assured inside; a conduit (313) is placed along a longitudinal direction and exactly at the basis of the surface in said cylindric balloon (314),
so that, it is possible to insert the catheter by sliding it in a endovascular way, following the guiding wire (311); when the catheter has reached its final position in the artery, by inflating the cylindric balloon (314) it is achieved the result to dilate and keep a sufficient stiffness into the catheter-blood vessel system, so that a proper blood flow is maintained inside the artery.

4. Surgical system connecting vascular segments that are not functionally contiguous, according to one or more of the previous claim, **characterized in that** comprising:
- a needle containing inside a thin metallic guiding wire (312), having few tenths of a millimeter thickness, placed at the position of said cylindric balloon (314) and able to move forward radially, puncture and pass through the walls of the artery.

5. Process to achieve a by-pass connecting vascular segments that are not functionally contiguous, using a surgical system as defined by one or more of the previous claims 1-5, comprising the following steps:
- a first endovascular catheter (207) is inserted under fluoroscopic guidance in the first artery (203) that has to be connected - i.e. the left anterior descending artery (LAD); a first cylindric balloon, placed at the distal end of said first endovascular catheter (207), is inflated so that it can adhere to the internal walls of the artery (203), being hollow inside and keeping therefore the normal blood flow;
- a second endovascular catheter (208) is inserted under fluoroscopic guidance in the first artery (204) that has to be connected - i.e. the left internal mammary artery (LIMA); a second cylindric balloon, placed at the distal end of said second endovascular catheter (208), is inflated so that it can adhere to the internal walls of the artery (204), being hollow inside and keeping therefore the normal blood flow;
- a first needle goes out from the first catheter (207), at a position close to the first cylindric balloon, in order to provide a puncture and pass through the wall of the first artery; a first guiding wire having a thickness of few tenths of a millimeter is pushed going through the inner lumen of the needle, so that the first wire is in an extravascular position;
- a second needle goes out from the second catheter (208), at a position close to the second cylindric balloon, in order to provide a puncture and pass through the wall of the second artery; a second guiding wire having a thickness of few tenths of a millimeter is pushed going through the inner lumen of the needle, so that the second wire is in an extravascular position;
- the distal ends of the first and second guiding wires (211, 212) are attracted and connected each other in order to achieve a unique wire working as a track;
- one of the two guiding wires, i.e. the guiding wire (212) is pulled and completely extracted, pulling with itself the end of the other guiding wire (211), so that only the latter one is maintained, representing a track;
- both the catheters (207) and (208) are removed;
- a stent (213) is positioned and installed by using the guiding wire (211), so that a by-pass is achieved connecting the left anterior descending artery (LAD) (203) to the left internal mammary artery (LIMA) (204).
